(19) Europäisches Patentamt
European Patent Office
Office européen des brevets

(11) EP 4 541 793 A1

(12) EUROPEAN PATENT APPLICATION
published in accordance with Art. 153(4) EPC

(43) Date of publication:
**23.04.2025 Bulletin 2025/17**

(21) Application number: **23822804.3**

(22) Date of filing: **26.04.2023**

(51) International Patent Classification (IPC):
***C07D 409/14*** *(2006.01)*

(52) Cooperative Patent Classification (CPC):
**C07C 51/41; C07C 51/43; C07C 57/15; C07D 409/14**

(86) International application number:
**PCT/CN2023/090820**

(87) International publication number:
**WO 2023/241233 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.06.2022 CN 202210670411**

(71) Applicant: **Fujian Mindong Rejuenation Pharmaceutical Co., Ltd.**
**Ningde, Fujian 355300 (CN)**

(72) Inventors:
- **LAM, Yuen**
**Ningde, Fujian 355300 (CN)**
- **LI, Lin**
**Ningde, Fujian 355300 (CN)**
- **YANG, Min**
**Ningde, Fujian 355300 (CN)**
- **LIN, Pengfei**
**Ningde, Fujian 355300 (CN)**
- **ZHANG, Yashan**
**Ningde, Fujian 355300 (CN)**
- **SONG, Han**
**Ningde, Fujian 355300 (CN)**
- **WANG, Haiyan**
**Ningde, Fujian 355300 (CN)**

(74) Representative: **Schiweck Weinzierl Koch Patentanwälte Partnerschaft mbB**
**Ganghoferstraße 68 B**
**80339 München (DE)**

# (54) RUPATIFEN FUMARATE CRYSTAL FORM C, AND PREPARATION METHOD THEREFOR AND USE THEREOF

(57) The present invention provides a rupatifen fumarate crystal form C, and a preparation method therefor and the use thereof. By using Cu-Kα radiation, characteristic peaks of the X-ray powder diffraction pattern of the crystal form C of the present invention, as represented by 2θ(±0.2°), are located at 12.3°±0.2°, 14.4°±0.2°, 17.6°±0.2° and 22.4°±0.2°. The crystal form C has the characteristics of a high melting point, a low amount of residual moisture, a low amount of residual ethanol and a more stable crystal form; few types of residual solvents and higher safety; using a single solvent and a low amount of solvent, and effectively saving material costs; and low environmental pollution, a high yield, mild conditions, and simple operation, and being more beneficial for later commercial production, etc.

EP 4 541 793 A1

## Description

### Technical Field of the Invention

The invention belongs to the technical field of pharmaceutical chemistry, and particularly relates to a crystalline form C of rupatifene fumarate, preparation method and use thereof.

### Background Art

WO2013000406 discloses a multi-channel drug for comprehensively inhibiting allergy, rupatifene fumarate, namely 4-[1- [(5-methylpyridin-3-yl) methyl]piperidyl-4-ylidene]-4, 9-dihydro-10H-benzo[4,5]cycloheptyl[1,2-b]thiophen-10-one fumarate, the structural formula is shown as follows:

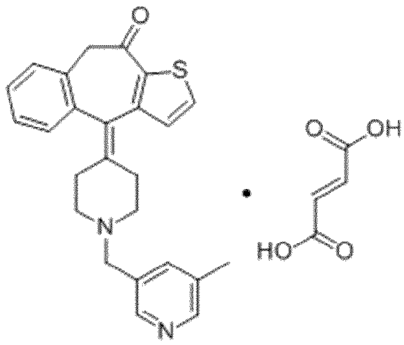

Drug polymorphism refers to the existence of two or more different crystalline forms of drugs. Polymorphism exists widely in drugs. Different crystalline forms of the same drug have significant differences in solubility, melting point, density, stability and the like, thereby affecting the stability, uniformity, bioavailability, curative effect and safety of the drug to different degrees. Therefore, it is one of the important contents that cannot be ignored to conduct a comprehensive and systematic polymorphic screening in drug research and development to select the most suitable crystalline form.

Rupatifene fumarate is polymorphic. CN104045633B discloses a crystalline form A of rupatifene fumarate, its X-ray powder diffraction pattern has characteristic peaks at the following 2θ(±0.2°)angles: 6.9°, 9.1°, 11.4°, 12.2°, 13.6°, 15.1°, 16.9°, 18.1°, 18.6° , 20.3°, 21.3°, 23.2°, 24.0°, 24.9°, 25.8°, 27.1°, 28.2°, 29.1°. CN 104059056B discloses a crystalline form B of rupatifene fumarate, its X-ray powder diffraction pattern has characteristic peaks at the following 2θ (±0.2°)angles: 5.9 °, 6.7 °, 8.0 °, 11.7 °, 12.0 °, 12.9 °, 14.5 °, 16.7 °, 17.2 °, 18.9 °, 19.7 °, 20.2 °, 22.3 °, 24.4 °, 25.9 °, 27.0 °, 28.8 ° and 30.7 °. Organic solvents such as dichloromethane are used in the preparation of these two crystalline forms, which will cause environmental pollution to a certain extent. At the same time, the yield is relatively low because of the constant temperature reduction.

CN 104031035B discloses a mixed crystal of rupatifene fumarate.

However, the above crystalline forms have low stability, low purity, large water residue, low melting point, and a large amount of residual solvent in the preparation process, resulting in low safety and high cost, and therefore they cannot be commercialized.

### Summary of the Invention

In order to overcome the defects of the prior art, the present invention provides a crystalline form C of rupatifene fumarate and preparation method thereof. The crystalline form C of rupatifene fumarate is more stable, with lower solvent residue, less moisture, and higher purity. The preparation method of the crystalline form is simple, easy to realize industrialization, and can ensure the quality and the medication safety of the new rupatifene fumarate drug.

In order to achieve the above object of the present invention, the following technical solutions are adopted.

In one aspect, the invention provides a crystalline form C of rupatifene fumarate, the X-ray powder diffraction pattern of the crystalline form C has characteristic peaks expressed in degrees 2θ (±0.2°) at 12.3 ° ± 0.2 °, 14.4 ° ± 0.2 °, 17.6 ° ± 0.2 °, 22.4 ° ± 0.2 ° using Cu-Ka radiation.

Preferably, the X-ray powder diffraction pattern of the crystalline form C has characteristic peaks expressed in degrees 2θ (±0.2°) at 8.7°±0.2°, 10.0°±0.2°, 12.3°±0.2°, 14.4°±0.2°, 17.6°±0.2°, 22.4°±0.2° using Cu-Ka radiation.

Preferably, the X-ray powder diffraction pattern of the crystalline form C has characteristic peaks expressed in degrees 2θ (±0.2°) at 8.7°±0.2°, 10.0°±0.2°, 12.3°±0.2°, 14.4°±0.2°, 17.6°±0.2°, 21.2°±0.2°, 22 .4°±0.2°, 24.1°±0.2° using Cu-Ka radiation.

Preferably, the X-ray powder diffraction pattern of the crystalline form C expressed in degrees 2θ (±0.2°) using Cu-Ka radiation is shown in figure 1.

Preferably, the melting point of the crystalline form C is 204.0 °C to 212.0 °C.

Preferably, the crystalline form C is spheroidal under microscopic conditions.

In another aspect, the invention provides a preparation method of the crystalline form C of rupatufen fumarate, comprising the following steps:
dissolving rupatifene free base and fumaric acid in a solvent A, stirring, heating and refluxing until the solution is clear, reacting, or dissolving crude rupatifene fumarate in the solvent A, heating, stirring and refluxing to prepare a saturated solution, then cooling to 2-8 °C, stirring for crystallization, filtering, washing with the solvent A, and drying under reduced pressure to obtain the crystalline form C of rupatufen fumarate.

Preferably, the solvent A is selected from ethanol aqueous solution (mass ratio) with an ethanol concentration not less than 95%, anhydrous ethanol solution, ethyl acetate-acetone solution, dichloromethane-acetone solution, ether-methanol solution, cyclohexane-ethyl acetate solution and other solvents for crystallization, and preferably anhydrous ethanol solution.

Preferably, the mass ratio of ethyl acetate to acetone in the ethyl acetate-acetone solution is 2: 1-1: 4 (w/w).

Preferably, the mass ratio of dichloromethane to acetone in the dichloromethane-acetone solution is 1: 1-1: 10 (w/w).

Preferably, the mass ratio of ether to methanol in the ether-methanol solution is 1: 1-1: 5 (w/w).

Preferably, the mass ratio of cyclohexane to ethyl acetate in the cyclohexane-ethyl acetate solution is 1: 1-1: 10 (w/w).

Preferably, the mass ratio of the rupatifene free base to the fumaric acid is 4:1 to 1: 2.

Preferably, the temperature for stirring, heating and refluxing until the solution is clear is 30-90 °C, and preferably 70 °C.

Preferably, the cooling is natural cooling, gradient cooling or rapid cooling.

When the temperature at which the solution is clear is higher than 40 °C, the procedure of the gradient cooling is as follows: cooling to 40 °C at a speed of 15±5°C/h; cooling to 15 °C at a speed of 7.5±2.5°C/h and keeping the temperature for 2 h; cooling to 5±3 °C at a speed of 7.5±2.5 °C /h and keeping the temperature for 2 h.

When the temperature at which the solution is clear is lower than 40 °C, the procedure of the gradient cooling is as follows: cooling to 15 °C at a speed of 7.5±2.5 °C/h and keeping the temperature for 2 h; cooling to 5±3 °C at a speed of 7.5 ±2.5 °C/h and keeping the temperature for 2 h.

Preferably, the speed of the stirring for crystallization is 50 to 200rmp, preferably 90 to 110rpm.

Preferably, the mass-to-volume ratio of the rupatufen free base to the solvent A or the crude rupatufen fumarate to the solvent A is from 2:1 to 1:30, preferably from 1:15 to 17.5.

In yet another aspect, the present invention provides a pharmaceutical composition comprising a therapeutically effective amount of the crystalline form C and optionally a pharmaceutically acceptable carrier or excipient.

In yet another aspect, the present invention provides a use of the crystalline form C in the preparation of a medicament for the treatment of an allergic disease or disorder;

Preferably, the allergic disease or disorder comprises: allergy, drug hypersensitivity, skin allergy, eczema, allergic rhinitis, urticaria, atopic dermatitis, dry eye, allergic contact allergy, food hypersensitivity, allergic conjunctivitis, insect venom allergy, bronchial asthma, allergic asthma, endogenous asthma, occupational asthma, ectopic asthma, acute respiratory distress syndrome (ARDS), and chronic obstructive pulmonary disease (COPD).

In yet another aspect, the present invention provides a method for treating an allergic disease or disorder, comprising administering the crystalline form C or the pharmaceutical composition to a patient in need;

Preferably, the allergic disease or disorder comprises: allergy, drug hypersensitivity, skin allergy, eczema, allergic rhinitis, urticaria, atopic dermatitis, dry eye, allergic contact allergy, food hypersensitivity, allergic conjunctivitis, insect venom allergy, bronchial asthma, allergic asthma, endogenous asthma, occupational asthma, ectopic asthma, acute respiratory distress syndrome (ARDS), and chronic obstructive pulmonary disease (COPD).

Compared with the prior art, the present invention at least has the following beneficial effects:
The invention provides a crystalline form C of rupatifene fumarate, the crystalline form C is high in purity, more stable, higher in melting point (reaching 204.0-212.0°C), and less in moisture and residual solvent. The preparation method of the crystalline form C has the advantages of high product yield, mild conditions, environment-friendly reagents, simple operation and easy industrialization.

Compared with the disclosed crystalline forms (including the crystalline form A, the crystalline form B and the mixed crystal), the crystalline form C has the advantages of high melting point, less moisture residue, more stable crystal form, high solubility, small hygroscopicity, good fat solubility and the like; meanwhile, it has less residual ethanol, less kinds of residual solvents and higher safety. From the analysis of process feasibility, the crystalline form A and the crystalline form B both adopt mixed solvents, the crystalline form C adopts a single solvent with less solvent consumption, and which can effectively save material costs. The use of organic solvents such as dichloromethane in the crystalline form A and the

crystalline form B will cause environmental pollution to a certain extent, while the use of anhydrous ethanol in crystalline form C has little environmental pollution. The yield of the preparation method of the crystalline form C is obviously higher than that of other crystal forms, and which is more favorable for the commercial production in the later period.

## Brief Description of Drawings

The invention will be further described with reference to the accompanying drawings.

Figure 1 is a powder diffraction pattern of the crystalline form C of rupatifene fumarate;
Figure 2 is a DSC profile of the crystalline form C of rupatifene fumarate;
Figure 3 is a TGA profile of the crystalline form C of rupatifene fumarate;
Figure 4 is a 100-fold microscopic picture of the crystalline form C of rupatifene fumarate.

## Detailed Description

The present invention will be illustrated below with reference to specific examples. It will be understood by those skilled in the art that these examples are merely illustrative and do not limit the scope of the present invention in any way.

Unless otherwise specified, the experimental procedures in the following examples are all conventional. Unless otherwise specified, the raw materials and reagent materials and the like used in the following examples are all commercially available products. The conditions of some instruments are as follows:
Instruments: a constant temperature magnetic stirrer (HWCL-5, Great Wall Science and Industry), a rotary evaporator (IKA), a blast drying box (DHG-9070, Shanghai Sanfa Scientific Instrument), a spherical condenser and several eggplant-shaped bottles (500 ml).

## Example 1

1.0g of rupatifene free base and 0.29g of fumaric acid were dissolved in 15ml of anhydrous ethanol, heated to 70-80 °C, stirred and refluxed until the sample were completely dissolved, to prepare a saturated solution, reacted for 1h at a rotating speed of 100rpm, and cooled to 40 °C at a speed of 15±5°C/h; and then cooled to 15 °C at a speed of 7.5±2.5 °C /h, under the temperature kept for 2h, cooled to 5±3 °C at a speed of 7.5±2.5 °C/h, and under the temperature kept for 2 h. A spheroidal crystal was precipitated, washed with anhydrous ethanol at 2°C ~8°C, and dried under reduced pressure at 60 °C to obtain the product.

## Example 2

1.0g of rupatifene free base and 0.29g of fumaric acid were dissolved in 15ml of anhydrous ethanol, heated, stirred and refluxed at 70 °C until the sample is completely dissolved, to prepare a saturated solution, reacted for 1h, naturally cooled to 50-60 °C, and continuously stirred to room temperature for crystallization after adding seed crystals, and then under the temperature of 2-8 °C kept for 2h. A spheroidal crystal was precipitated, washed with anhydrous ethanol at 2°C~8°C, and dried under reduced pressure at 60 °C to obtain the product.

## Example 3

1.0g of crude rupatifene fumarate was dissolved in 15ml of anhydrous ethanol, heated to 70-80 °C, stirred and refluxed until the sample was completely dissolved to prepare a saturated solution, reacted for 1h at a rotating speed of 100rpm, and cooled to 40 °C at a speed of 15±5°C/h; cooled to 15 °C at a speed of 7.5±2.5°C/h, under the temperature kept for 2h, cooled to 5±3 °C at a speed of 7.5±2.5 °C/h, and under the temperature kept for 2 h. A spheroidal crystal was precipitated, washed with anhydrous ethanol at 2°C~8°C, and dried under reduced pressure at 60 °C to obtain the product.

## Example 4

2.0g of crude rupatifene fumarate was dissolved in 35ml of anhydrous ethanol solution, heated, stirred and refluxed until the sample was completely dissolved to prepare a saturated solution, naturally cooled to room temperature, and then cooled to 2-8 °C for crystallization, stirred to separate out spheroidal crystals, washed with 2-8 °Canhydrous ethanol solution, and dried under reduced pressure at 60 °C to obtain the product.

**Example 5**

1.0g of rupatifene free base and 0.29g of fumaric acid were dissolved in 18ml of 95% ethanol (mass ratio), heated, stirred and refluxed at 70-80 °C until the sample was completely dissolved to prepare a saturated solution, reacted for 1h, and cooled to 40 °C at a speed of 15$\pm$5 °C/h; cooled to 15 °C at a speed of 7.5$\pm$2.5 °C/h, under the temperature kept for 2h, cooled to 5$\pm$3 °C at a speed of 7.5$\pm$2.5 °C/h, and under the temperature kept for 2 h, stirred to separate out spheroidal crystals, washed with 95% ethanol solution at 2-8 °C, and dried under reduced pressure at 60 °C to obtain the product.

**Example 6**

2.0g of crude rupatifene fumarate was dissolved in 40ml of 95% ethanol (mass ratio), heated, stirred and refluxed until the sample was completely dissolved to prepare a saturated solution, naturally cooled to room temperature, cooled to 2-8 °C for crystallization, stirred to separate out spheroidal crystals, washed with 2-8 °C 95% ethanol solution, and dried at 60 °C under reduced pressure to obtain the product.

**Example 7**

1.0g of rupatifene free base and 0.29g of fumaric acid were dissolved in 30ml of ethyl acetate-acetone (1: 1, w/w), heated, stirred and refluxed until the sample was completely dissolved to prepare a saturated solution, reacted for 0.5h, the saturated solution was cooled to 5 °C at a speed of 20-30 °C/h to precipitate spheroidal crystals, washed with ethyl acetate-acetone (1: 1, w/w) at 2-8 °C, and dried under reduced pressure at 60 °C to obtain the product.

**Example 8**

1.0g of crude rupatifene fumarate was dissolved in 25ml of ethyl acetate-acetone (1: 1, w/w), heated, stirred and refluxed until the sample was completely dissolved to prepare a saturated solution, reacted for 0.5h, the saturated solution was cooled to 5 °C at a speed of 20-30 °C/h to separate out spheroidal crystals, washed with ethyl acetate-acetone (1: 1, w/w) at 2-8 °C, and dried under reduced pressure at 60 °C to obtain the product.

**Example 9**

1.0g of rupatifene free base and 0.32g of fumaric acid were dissolved in 20ml of dichloromethane-acetone solution (1: 1, w/w), stirred and heated and refluxed until the sample was completely dissolved to prepare a saturated solution, reacted for 1h, naturally cooled to room temperature, cooled to 2-8 °C for crystallization, stirred to separate out spheroidal crystals, washed with 2-8 °C dichloromethane-acetone solution (1: 1, w/w), and dried at 60 °C under reduced pressure to obtain the product.

**Example 10**

1.0g of crude rupatifene fumarate was dissolved in 20ml of dichloromethane-acetone solution (1: 1, w/w), stirred, heated and refluxed until the sample was completely dissolved to prepare a saturated solution, reacted for 1h, naturally cooled to room temperature, cooled to 2-8 °C for crystallization, stirred to separate out spheroidal crystals, washed with dichloromethane-acetone solution (1: 1, w/w) solution at 2-8 °C, and dried under reduced pressure at 60 °C to obtain the product.

**Example 11**

3.5g of rupatifene free base and 1g of fumaric acid were dissolved in 70ml of diethyl ether-methanol solution (1: 1, w/w), heated, stirred and refluxed until the sample was completely dissolved to prepare a saturated solution, reacted for 1h, naturally cooled to room temperature, cooled to 2-8 °C for crystallization, stirred to separate out spheroidal crystals, washed with diethyl ether-methanol solution (1: 1, w/w) at 2-8 °C, and dried under reduced pressure at 60 °C to obtain the product.

**Example 12**

2.0g of crude rupatifene fumarate was dissolved in 40ml of diethyl ether-methanol solution (1: 1, w/w), heated, stirred and refluxed until the sample was completely dissolved to prepare a saturated solution, reacted for 1h, naturally cooled to room temperature, cooled to 2-8 °C for crystallization, stirred to separate out spheroidal crystals, washed with

diethyl ether-methanol solution (1: 1, w/w) at 2-8 °C, and dried under reduced pressure at 60 °C to obtain the product.

**Example 13**

1.0g of rupatifene free base and 0.32g of fumaric acid were dissolved in 30ml of cyclohexane-ethyl acetate solution (1: 1, w/w), heated, stirred and refluxed until the sample is completely dissolved to prepare a saturated solution, naturally cooled to room temperature, cooled to 2-8 °C for crystallization, stirred to separate out spheroidal crystals, washed with 2-8 °C cyclohexane-ethyl acetate solution (1: 1, w/w), and dried at 60 °C under reduced pressure to obtain the product.

**Example 14**

2.0g of crude rupatifene fumarate was dissolved in 60ml of cyclohexane-ethyl acetate solution (1: 1, w/w), heated, stirred and refluxed until the sample was completely dissolved to prepare a saturated solution, naturally cooled to room temperature, cooled to 2-8 °C for crystallization, stirred to separate out spheroidal crystals, washed with 2-8 °C cyclohexane-ethyl acetate solution (1: 1, w/w), and dried under reduced pressure at 60 °C to obtain the product.

**Example 15**

In this example, a crystalline form C was compared with a crystalline form A, a crystalline form B and a mixed crystal, wherein the crystalline form C was prepared from example 1, the crystalline form A was prepared from example 1 of Chinese patent CN 104045633B, the crystalline form B was prepared from example 1 of Chinese patent CN 104059056B, the mixed crystal was obtained from example 1 of Chinese patent CN 104031035B. The key process parameters, yield, physical and chemical properties, solvent residue and other data for preparing these crystal forms are listed in table 1.

1. Key process conditions

Table 1 Process conditions of different crystal forms of rupatifen fumarate

| Crystal Forms | Key Process Conditions | | | | Yield |
|---|---|---|---|---|---|
| | Solvent(s) | Solvent Ratio | Dissolution Temperature | Crystallization Mode | |
| Crystalline form A | Dichloromethane and methanol solution (1: 1) | 1:28 | Room temperature | Concentrated under reduced pressure to 25% -30%, naturally cooled to room temperature, stood at 2-8 °C for crystallization | 70%~75 % |
| Crystalline form B | Dichloromethane, 95% ethanol solution (1: 1) | 1:20 | Room temperature | Concentrated under reduced pressure to 25% -30%, naturally cooled to room temperature, stood at 2-8 °C for crystallization | 80%~85 % |
| Mixed crystals | Ethanol aqueous solution (95%) | 1:20 | Room temperature | Concentrated under reduced pressure to 25% -30%, naturally cooled to room temperature, stood at 2-8 °C for crystallization | 80%~85 % |
| Crystalline form C | Anhydrous ethanol | 1:15 | 70 °C ~80 °C | 1. Cooled to 40 °C at a speed of 15±5 °C/h, under the temperature kept for crystallization;<br>2. Cooled to 15 °C at a speed of 7.5±2.5 °C/h, under the temperature kept for 2h;<br>3. Cooled to 5±3 °C at a speed of 7.5±2.5 °C/h, under the temperature kept for 2h; | 85%~92 % |

The crystalline form A, the crystalline form B and the mixed crystal all adopt mixed solvents, the crystalline form C adopts a single solvent with a small amount of solvent, which can effectively save the material cost. Meanwhile, the use of

organic solvents such as dichloromethane in the crystalline form A and the crystalline form B will cause environmental pollution to a certain extent, while the use of anhydrous ethanol in the crystalline form C has little environmental pollution. The yield of the preparation method of the crystalline form C is obviously higher than that of other crystal forms, and which is more favorable for the commercial production in the later period.

2. Physical and chemical properties

The following analytical methods were used to test samples of different crystal forms, and the data are shown in tables 2-5.

2.1 Melting point determination method:

A sample was taken and placed in a capillary for melting point measurement. The powder was tightly concentrated at the melt-sealed end of the capillary by 3 mm. The preset temperature was 190 °C, and the heating rate was 3 °C/min. The temperature of the test sample from initial melting to full melting was recorded, the measurement was repeated 3 times, and the average value was taken as the result.

2.2 Moisture determination method:

A sample of 0.8g was taken and measured according to a moisture determination method (China Pharmacopoeia, 2020 Edition, General Rules 0832, First Method 1). The specific operation was as follows:

1) Blank calibration: the moisture analyzer was calibrated to be in an anhydrous state;
2) Calibration: 10mg of purified water was precisely weighed into the analyzer in an anhydrous state for measurement, and the titer was calculated. The measurement was performed for 3 times, and the calculated RSD shall not exceed 2.0%;
3) Test sample determination: the test sample not less than 0.8g (based on the sample weight calculated by the actual measured F value of commercial Fisher's test solution) was taken into the analyzer in an anhydrous state for measurement. The measurement was performed continuously for 3 times, and the average value was taken as the result.

2.3 Crystal habit detection method

An appropriate amount of sample was taken, dispersed with cyclohexane and observed under a microscope.

2.4 Fluidity detection method:

Measurement of angle of repose: a fixed cone bottom method was used, the funnel was fixed on a horizontal coordinate paper at a proper height, so that the distance from a lower opening of the funnel to the coordinate paper was H, the powder was carefully poured into the funnel until the tip of a cone formed below the funnel contacted an outlet of the funnel, the radius r of the cone can be measured by the coordinate paper, and measured three times continuously, and the average value was taken as the result. The angle of repose $\alpha$ was calculated as follows:

$$\alpha = \tan\text{-}1\ (H/r).$$

Judgment standard: when the angle of repose is < 30° , the fluidity is better. When the angle of repose is > 40° , the fluidity is poor.

2.5 Analysis method of hygroscopicity:

1) a dry glass weighing bottle with a plug (with an outer diameter of 50mm and a height of 15mm) was taken and placed in a proper constant-temperature dryer (the lower part was placed with ammonium chloride or ammonium sulfate saturated solution) at 25°C±1°C one day before the test; and the weight of the weighing bottle was precisely measured (m 1).
2) a proper amount of the test sample was taken, tiled in the weighing bottle, wherein the thickness of the test sample

was about 1mm, and the weight of the test sample was precisely measured (m 2). 3) the weighing bottle was opened, and placed with its bottle cap under the constant temperature and humidity conditions for 24 hours.
4) the bottle cap was covered well and the weight of the bottle cap was precisely measured (m 3).

$$\text{Weight gain percent} = (m\ 3 - m\ 2)/(m\ 2 - m\ 1) \times 100\%$$

According to 9103 guideline principle on drug hygroscopicity test in four part of Chinese Pharmacopoeia 2020 edition, the description of hygroscopicity characteristics and the definition of hygroscopicity weight gain are as follows: Deliquescence: enough water is absorb to form liquid.
Extremely hygroscopic: the weight gain due to hygroscopicity is not less than 15%.
Hygroscopic: the weight gain due to hygroscopicity is less than 15% but not less than 2%.
Slightly hygroscopic: the weight gain due to hygroscopicity is less than 2% but not less than 0.2%.
No or almost no hygroscopicity: the weight gain due to hygroscopicity is less than 0.2%.

2.6 Analysis method of solubility:

The excessive test sample was put into a dissolution instrument cup, added with water and an appropriate amount of water-soluble medium with different pH values (pH =1.2, 2.2, 4.0, 4.5, 5.5, 6.8), stirred at 37°C ±1 °C (rotation speed: 100 r/min) for dissolution (more than 6 hours) to form a supersaturated solution, the saturated solution was filtered, the filtrate was diluted with a mobile phase by corresponding times, the concentration was measured by HPLC method, and the solubility was calculated.

2.7 Analysis method of oil-water distribution coefficient:

The standard substance having known log P (n-octanol-water) was used, the log P (n-octanol-water) of the substance to be tested was detected by HPLC. The principle is based on the fact that the retention time of chemical substances in a long hydrocarbon chain chromatographic column such as C18 column is related to their hydrocarbon-water distribution coefficient. Hydrophilic substances are eluted first, lipophilic substances are eluted later, and the oil-water distribution coefficient and the retention time are in a linear relationship, and the equation is as follows:

$$\log P = a \log K + b$$

$$K = (T_n - T_0)/T_0.$$

log P: oil-water partition coefficient of samples (n-octanol-water); $T_0$: dead time (measured by uracil here); $T_n$: retention time of samples by HPLC; according to the above formula, a linear relationship formula can be established by detecting the retention time of the standard substance and the known log P (n-octanol-water). The theoretical value of log P is used as the ordinate, the log K value is used as the abscissa for drawing, the values of a and b are obtained through calculation, and then the retention time (namely the logK value in the table) of samples to be detected is substituted into the equation to calculate the log P (n-octanol-water) value of samples.

2.8 results

The results of the above analysis methods are shown in tables 2 to 5. Table 2 shows the physical and chemical properties of different crystal forms, table 3 shows the results of hygroscopicity, table 4 shows the solubility data, and table 5 shows the results of oil-water distribution coefficient.

Table 2 Physical and chemical properties of different crystal forms of rupatifene fumarate

| Crystalline Form | Characteristics | Melting point | Moisture | Crystal Habit | Angle of Repose |
|---|---|---|---|---|---|
| Crystalline form A | White-like powder | 152°C~ 156°C | 3.0% | Crystalline form is rod-shaped | $47^0$ |
| Crystalline form B | White-like powder | 184°C~ 188°C | 2.4% | Crystalline form is rod-shaped | $50^0$ |
| Mixed crystal | White-like powder | 152°C~ 156°C | 2.6% | Crystalline form is rod-shaped | $49^0$ |

(continued)

| Crystalline Form | Characteristics | Melting point | Moisture | Crystal Habit | Angle of Repose |
|---|---|---|---|---|---|
| Crystalline form C | White-like powder | 204°C~ 212°C | 0.55% | Crystalline form is spherical | $28^0$ |

As shown in table 2, the crystalline form C has a higher melting point and less water than other crystal forms, so it can be inferred that the possibility that the crystalline form C contains crystal water is small, and the raw material of the crystalline form C tends to be a more stable crystal form; on the other hand, as the crystal form of the crystalline form C is spherical, other crystal forms are rod-shaped, and the angle of repose of the crystalline form C is less than 30°, and the fluidity of the crystalline form C is better, so that it is more conducive to the development of rupatifen fumarate capsule preparations with an uniform content.

Table 3 Results of hygroscopicity

| Crystalline Form | Weight Gain | Hygroscopicity |
|---|---|---|
| Crystalline form B | 0.2% | Slightly hygroscopic |
| Crystalline form C | 0.06% | No or almost no hygroscopicity |
| Crystalline form A | 0.3% | Slightly hygroscopic |
| Mixed crystal | 0.4% | Slightly hygroscopic |

As shown in table 3, the crystalline form C has slightly better hygroscopicity than other crystal forms, has less influence by water molecules during the storage and use of active pharmaceutical ingredients, which is more favorable for ensuring the stability of active pharmaceutical ingredients during the storage and use.

Table 4 Results of solubility

| Crystalline Form | Medium | Concentration (mg/ml) | Equilibrium Solubility (mg/250ml) |
|---|---|---|---|
| Crystalline form B | pH=1.2 | 29.42 | 7355.56 |
| | pH=2.2 | 2.64 | 659.74 |
| | pH=4.0 | 1.41 | 352.76 |
| | pH=4.5 | 1.36 | 339.48 |
| | pH=5.5 | 0.18 | 44.97 |
| | pH=6.8 | 0.01 | 2.56 |
| | Water for injection | 1.22 | 305.45 |
| Crystalline form C | pH=1.2 | 19.64 | 4908.73 |
| | pH=2.2 | 4.05 | 1013.35 |
| | pH=4.0 | 2.84 | 710.90 |
| | pH=4.5 | 3.77 | 942.80 |
| | pH=5.5 | 0.31 | 77.90 |
| | pH=6.8 | 0.018 | 4.40 |
| | Water for injection | 2.46 | 616.34 |

(continued)

| Crystalline Form | Medium | Concentration (mg/ml) | Equilibrium Solubility (mg/250ml) |
|---|---|---|---|
| Crystalline form A | pH=1.2 | 26.68 | 6669.86 |
| | pH=2.2 | 3.00 | 751.00 |
| | pH=4.0 | 2.29 | 571.88 |
| | pH=4.5 | 2.99 | 747.72 |
| | pH=5.5 | 0.34 | 84.93 |
| | pH=6.8 | 0.01 | 3.39 |
| | Water for injection | 1.79 | 448.75 |
| Mixed crystal | pH=1.2 | 24.47 | 6117.84 |
| | pH=2.2 | 3.40 | 849.73 |
| | pH=4.0 | 2.53 | 633.71 |
| | pH=4.5 | 3.43 | 856.32 |
| | pH=5.5 | 0.31 | 76.85 |
| | pH=6.8 | 0.01 | 3.61 |
| | Water for injection | 1.98 | 495.71 |

As shown in table 4, according to the classification system of biopharmaceuticals, under the conditions of pH =1.2, pH =2.2, pH =4.0, pH =4.5, pH =5.5, and aqueous medium, different crystal forms have high solubility according to the requirements of formulation development; however, under the medium with pH=6.8, different crystal forms have no high solubility, wherein the solubility of the crystalline form C is slightly superior to that of other crystal forms. Therefore, the crystalline form C is considered to have slightly superior solubility properties compared with other crystal forms.

Table 5 Results of oil-water distribution coefficient

| Crystalline Form | log P(n-octanol-water) |
|---|---|
| Crystalline form B | 2.9 |
| Crystalline form C | 3.2 |
| Crystalline form A | 2.8 |
| Mixed crystal | 2.7 |

As shown in Table 5, the oil-water distribution coefficient of the crystalline form C is slightly better than that of other crystal forms, and the crystalline form C has better fat solubility, which indicates that it is easier to be absorbed in human body and has curative effect.

3. Solvent residue

A proper amount of sample was taken, precisely weighed, added with N, N-dimethylformamide to dissolve to prepare a solution containing about 50mg of N, N-dimethylformamide per 1 ml. A proper amount of methanol, n-pentane, ethanol, dichloromethane and ethyl acetate were taken, precisely weighed, and quantitatively diluted by adding N, N-dimethylformamide to prepare a mixed solution containing 150 μg of methanol, 250μg of n-pentane, 500μg of ethanol, 30μg of dichloromethane and 250μg of ethyl acetate per ml. A capillary column with 6% cyanopropyl phenyl-94% dimethyl polysiloxane (or similar polarity) as the stationary phase was used as the chromatographic column, the initial temperature was 30 °C, which was maintained for 4 minutes, the temperature was raised to 130 °C at a rate of 20 °C per minute, which was maintained for 3.5 minutes. The inlet temperature was 200 °C, and the detector temperature was 250 °C. The sample volume was 1μl. In the chromatogram of the reference solution, the separation degree between the peaks of each component should be greater than 1.5. The test solution and the reference solution were precisely measured, respectively injected into a gas chromatograph, and the chromatogram was recorded.

Table 6 Residual solvents of rupatifene fumarate in different crystal forms

| Crystalli ne Form | Methan ol Residue (%) | n-Pentan e Residu e (%) | Ethano l Residu e (%) | Aceto ne Residu e (%) | Dichlorometh ane Residue (%) | Ethyl Acetat e Residu e (%) |
|---|---|---|---|---|---|---|
| Crystalli ne Form A | 0.41 | Not detecte d | Not detecte d | Not detecte d | 0.12 | Not detecte d |
| Crystalli ne Form B | 0.04 | Not detecte d | 0.50 | 0.006 | 0.05 | Not detecte d |
| Mixed crystal | N/A | Not detecte d | 0.42 | Not detecte d | Not detected | Not detecte d |
| Crystalli ne Form C | Not detecte d | Not detecte d | 0.16 | N/A | Not detected | Not detecte d |
| Note: N/A means "not applicable". | | | | | | |

As shown in table 6, the crystal forms are compared, the crystalline form A is dissolved by methanol and dichloromethane, the methanol belongs to the second kind of dissolution, and the residual methanol is not beneficial to the preparation of medicine. The crystalline form B is dissolved by dichloromethane ethanol solution, and all of solvents belong to three kinds of solvents. Because the mixed solvent is used for crystallization in the process, the risk of residual solvent in the finished product is increased, the residues of two solvents need to be controlled to the limit requirement, which is less than 0.5%. The crystalline form C uses anhydrous ethanol, which belongs to three solvents, which should less than the limit requirement, which is less than 0.5%, wherein the residual ethanol is obviously less than that of other crystal forms, the types of residual solvents are less than that of other crystal forms (crystalline form A, crystalline form B and mixed crystal). It can be inferred that the possibility that a product contains crystallization solvent is small. In conclusion, the crystalline form C of rupatifen fumarate is safer.

**Example 16**

1.0g of rupatifene free base and 0.29g of fumaric acid were dissolved in 15ml of absolute ethanol, heated to 70-80 °C, stirred and refluxed until the sample was completely dissolved to prepare a saturated solution, and reacted for 1 hour at a rotating speed of 100 rpm. The cooling modes were as follows: 1. cooling to 40 °C at a speed of 15±5°C /h; cooling to 15 °C at a speed of 7.5±2.5°C/h, under the temperature keeping for 2h, cooling to 5±3°C at a speed of 7.5±2.5°C/h, and under the temperature keeping for 2 h; 2. cooling to 40 °C at a rate of 20-30°C /h, cooling to 15°C at a rate of 10-20 °C/h, under the temperature keeping for 2h, and cooling to 2-8°C at a rate of 10-20°C/h; 3. naturally cooling to room temperature, and then cooling to 2-8 °C; 4. cooling to 2-8 °C at a speed of 20-30 °C/h. Spherical crystals were precipitated, washed with 2-8 °C anhydrous ethanol solution, dried under reduced pressure at a temperature of 60 °C to obtain a crystalline form C. The solvent residue and water were detected respectively.

Table 7 Experimental results of different cooling modes

| Nos. | Cooling Modes | Detection Item | |
|---|---|---|---|
| | | Ethanol Residue (%) | Moisture (%) |
| 1 | Cooling to 40 °C at a speed of 15±5°C/h; cooling to 15 °C at a speed of 7.5 ±2.5°C/h, under the temperature keeping for 2h, cooling to 5±3°C at a speed of 7.5±2.5°C/h, and under the temperature keeping for 2 h | 0.16 | 0.55 |
| 2 | Cooling to 40 °C at a rate of 20-30°C/h, cooling to 15°C at a rate of 10-20°C/h, under the temperature keeping for 2h, and cooling to 2-8°C at a rate of 10-20°C/h | 0.21 | 0.89 |
| 3 | Naturally cooling to room temperature, and then cooling to 2-8 °C | 0.28 | 0.91 |
| 4 | Cooling to 2-8 °C at a speed of 20-30 °C/h | 0.33 | 1.02 |

The experimental research shows that the cooling speed should not be too fast, and the accelerated cooling will lead to the rapid crystallization, which will not enter a stable state. The residual solvent and water in the rapid cooling group

are higher than those in the gradient cooling group, which indicates that the phenomenon of inclusion of solvent and water occurs.

**Example 17**

The crystalline form C of rupatifene fumarate obtained in example 1 was taken, ground, sampled for X-ray powder diffraction, wherein the radiation source was Cu-Ka, and the result is shown in figure 1.
The crystalline form C of rupatifene fumarate obtained in examples 2-14 were taken, ground, sampled for X-ray powder diffraction, wherein the radiation source was Cu-Ka, and the result is similar to that in figure 1.

**Example 18**

The crystalline form C of rupatifene fumarate obtained in example 1 was detected by differential scanning calorimetry (DSC), and the melting point was at about 200-220 °C, at which time it decomposed, as shown in figure 2.
The crystalline form C of rupatifene fumarate obtained in examples 2-14 were detected by differential scanning calorimetry (DSC), the melting point was at about 200-220 °C, at which time it decomposed, and the result is similar to that in figure 2.

**Example 19**

The crystalline form C of rupatifene fumarate obtained in example 1 was detected by thermogravimetric analysis (TGA), and the melting point was at about 200-220 °C, at which time it decomposed, as shown in figure 3.
The crystalline form C of rupatifene fumarate obtained in examples 2-14 were detected by thermogravimetric analysis (TGA), the melting point was at about 200-220 °C, at which time it decomposed, and the result is similar to that in figure 3.

**Example 20**

The crystalline form C of rupatifene fumarate obtained in example 1 was observed under a microscope of 100-fold, its shape was spheroidal, as shown in figure 4.
The crystalline form C of rupatifene fumarate obtained in examples 2-14 were observed under a microscope of 100-fold, their shape were spheroidal, and the results were similar to those in figure 4.

## Claims

1. A crystalline form C of rupatifene fumarate, the X-ray powder diffraction pattern of the crystalline form C has characteristic peaks expressed in degrees 2θ (±0.2°) at 12.3 ° ± 0.2 °, 14.4 ° ± 0.2 °, 17.6 ° ± 0.2 °, 22.4 ° ± 0.2 ° using Cu-Ka radiation.

2. The crystalline form C according to claim 1, wherein the X-ray powder diffraction pattern of the crystalline form C has characteristic peaks expressed in degrees 2θ (±0.2°) at 8.7°±0.2°,10.0°±0.2°,12.3°±0.2°,14.4°±0.2°,17.6°±0.2°,22.4°±0.2° using Cu-Ka radiation;

    Preferably, the X-ray powder diffraction pattern of the crystalline form C has characteristic peaks expressed in degrees 2θ (±0.2°) at 8.7°±0.2°,10.0°±0.2°,12.3°±0.2°,14.4°±0.2°,17.6°±0.2°,21.2°±0.2°,22.4°±0.2°,24.1°±0.2° using Cu-Ka radiation;
    Preferably, the X-ray powder diffraction pattern of the crystalline form C expressed in degrees 2θ (±0.2°) using Cu-Ka radiation is shown in figure 1.

3. The crystalline form C according to claim 1 or 2, wherein the melting point of the crystalline form C is 204.0 °C to 212.0 °C;
   Preferably, the crystalline form C is spheroidal under microscopic conditions.

4. A preparation method of the crystalline form C of rupatufen fumarate according to any one of claims 1 to 3, comprising the following steps:
   dissolving rupatifene free base and fumaric acid in a solvent A, stirring, heating and refluxing until the solution is clear, reacting, or dissolving crude rupatifene fumarate in the solvent A, heating, stirring and refluxing to prepare a saturated

solution, and then cooling to 2-8 °C, stirring for crystallization, filtering, washing with the solvent A, and drying under reduced pressure to obtain the crystalline form C of rupatufen fumarate.

**5.** The method according to claim 4, wherein the solvent A is selected from ethanol aqueous solution (mass ratio) with an ethanol concentration not less than 95%, anhydrous ethanol solution, ethyl acetate-acetone solution, dichloromethane-acetone solution, ether-methanol solution, cyclohexane-ethyl acetate solution and other solvents for crystallization, and preferably anhydrous ethanol solution.

**6.** The method according to claim 5, wherein the mass ratio of ethyl acetate to acetone in the ethyl acetate-acetone solution is 2: 1-1: 4 (w/w);

Preferably, the mass ratio of dichloromethane to acetone in the dichloromethane-acetone solution is 1: 1-1: 10 (w/w);
Preferably, the mass ratio of ether to methanol in the ether-methanol solution is 1: 1-1: 5 (w/w);
Preferably, the mass ratio of cyclohexane to ethyl acetate in the cyclohexane-ethyl acetate solution is 1: 1-1: 10 (w/w).

**7.** The method according to any one of claims 4 to 6, wherein the mass ratio of the rupatifene free base to the fumaric acid is 4:1 to 1: 2;
Preferably, the temperature for stirring, heating and refluxing until the solution is clear is 30-90 °C, and preferably 70 °C.

**8.** The method according to any one of claims 4 to 7, wherein the cooling is natural cooling, gradient cooling or rapid cooling;

When the temperature at which the solution is clear is higher than 40 °C, the procedure of the gradient cooling is as follows: cooling to 40 °C at a speed of $15\pm5$°C/h; cooling to 15 °C at a speed of $7.5\pm2.5$°C/h and keeping the temperature for 2 h; cooling to $5\pm3$°C at a speed of $7.5\pm2.5$ °C /h and keeping the temperature for 2 h;
When the temperature at which the solution is clear is lower than 40 °C, the procedure of the gradient cooling is as follows: cooling to 15 °C at a speed of $7.5\pm2.5$ °C/h and keeping the temperature for 2 h; cooling to $5\pm3$ °C at a speed of $7.5\pm2.5$ °C/h and keeping the temperature for 2 h.

**9.** The method according to any one of claims 4 to 8, wherein the speed of the stirring for crystallization is 50 to 200rmp, preferably 90 to 110rpm.

**10.** The method according to any one of claims 4 to 9, wherein the mass-to-volume ratio of the rupatufen free base to the solvent A or the crude rupatufen fumarate to the solvent A is from 2:1 to 1:30, preferably from 1:15 to 17.5.

**11.** A pharmaceutical composition comprising a therapeutically effective amount of the crystalline form C according to any one of claims 1 to 3 and optionally a pharmaceutically acceptable carrier or excipient.

**12.** Use of the crystalline form C according to any one of claims 1 to 3 in the preparation of a medicament for the treatment of an allergic disease or disorder;
Preferably, the allergic disease or disorder comprises: allergy, drug hypersensitivity, skin allergy, eczema, allergic rhinitis, urticaria, atopic dermatitis, dry eye, allergic contact allergy, food hypersensitivity, allergic conjunctivitis, insect venom allergy, bronchial asthma, allergic asthma, endogenous asthma, occupational asthma, ectopic asthma, acute respiratory distress syndrome (ARDS), and chronic obstructive pulmonary disease (COPD).

**13.** A method for treating an allergic disease or disorder, comprising administering the crystalline form C according to any one of claims 1 to 3 or the pharmaceutical composition according to claim 11 to a patient in need;
Preferably, the allergic disease or disorder comprises: allergy, drug hypersensitivity, skin allergy, eczema, allergic rhinitis, urticaria, atopic dermatitis, dry eye, allergic contact allergy, food hypersensitivity, allergic conjunctivitis, insect venom allergy, bronchial asthma, allergic asthma, endogenous asthma, occupational asthma, ectopic asthma, acute respiratory distress syndrome (ARDS), and chronic obstructive pulmonary disease (COPD).

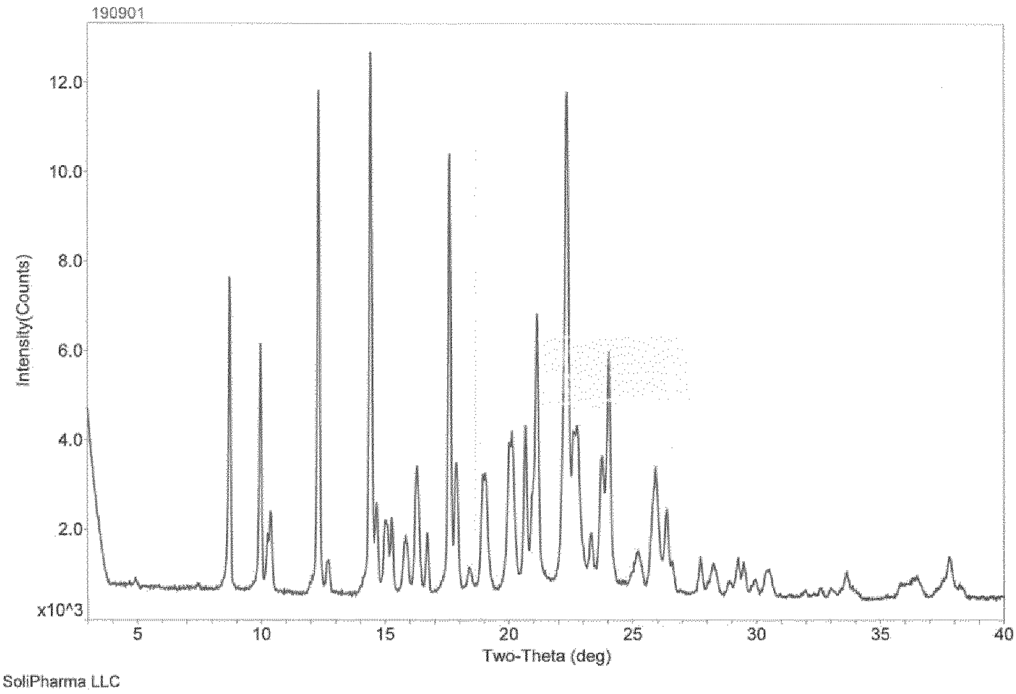


Figure 1

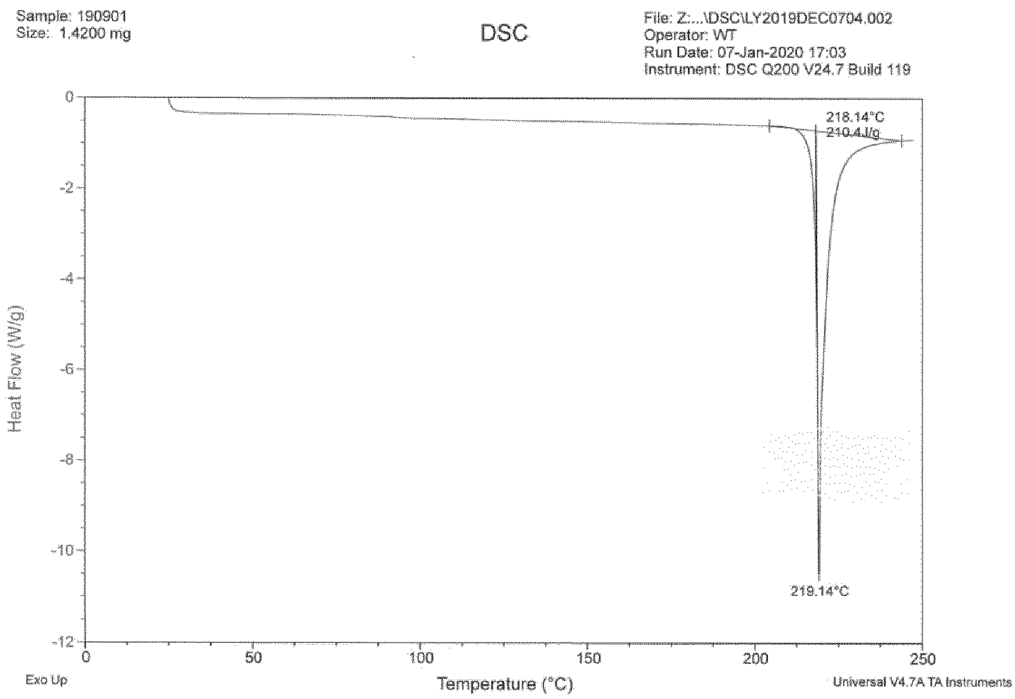


Figure 2

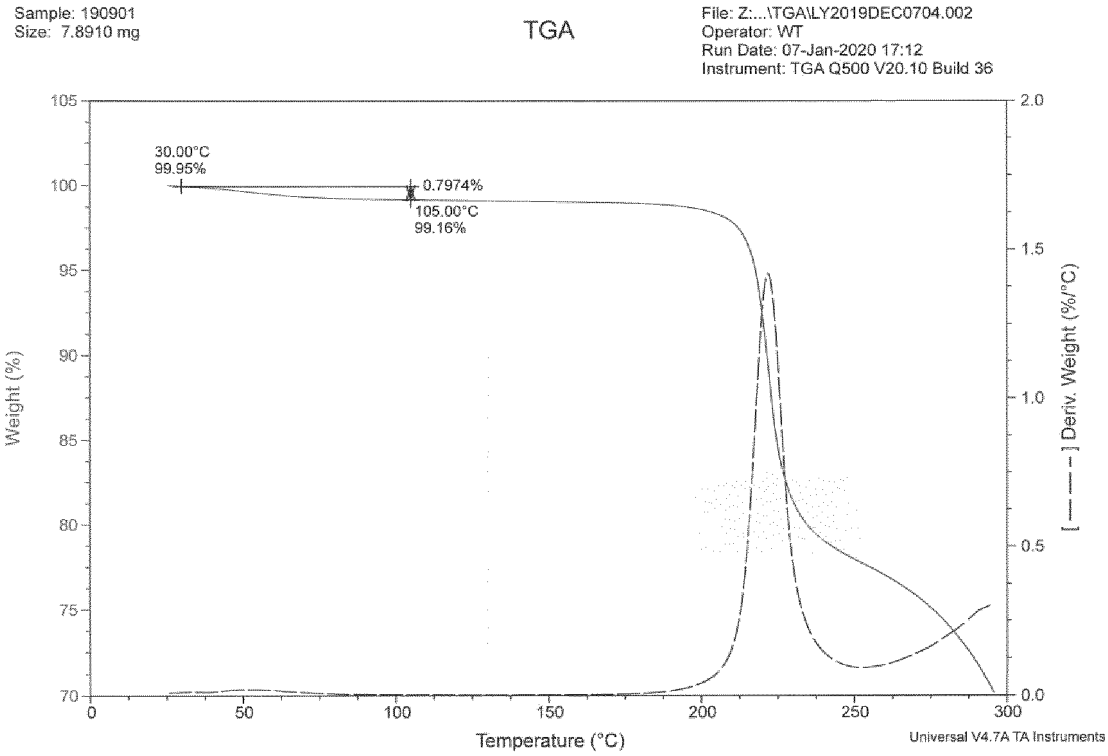


**Figure 3**

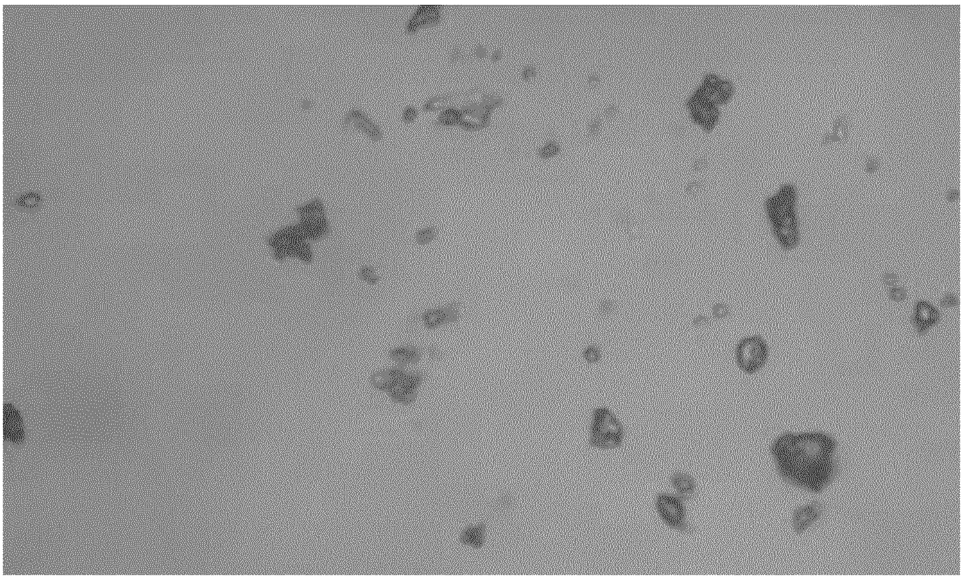

**Figure 4**

## INTERNATIONAL SEARCH REPORT

International application No.
**PCT/CN2023/090820**

**A. CLASSIFICATION OF SUBJECT MATTER**

C07D409/14(2006.01)i

According to International Patent Classification (IPC) or to both national classification and IPC

**B. FIELDS SEARCHED**

Minimum documentation searched (classification system followed by classification symbols)

C07D409/-

Documentation searched other than minimum documentation to the extent that such documents are included in the fields searched

Electronic data base consulted during the international search (name of data base and, where practicable, search terms used)

CNABS, CNTXT, VEN, ENTXT, CNKI, ISI, STN(Registry, Caplus): 富马酸, 卢帕替芬, 晶型, 苯并, 环庚, 噻吩, fumarate, rupatifen, lupatifen, crystal, 根据富马酸卢帕替芬结构式进行结构检索, search according to the structure of the structural formula of rupatifen fumarate

**C. DOCUMENTS CONSIDERED TO BE RELEVANT**

| Category* | Citation of document, with indication, where appropriate, of the relevant passages | Relevant to claim No. |
|---|---|---|
| A | CN 104031035 A (FUJIAN MINDONG REJUENATION PHARMACEUTICAL CO., LTD.) 10 September 2014 (2014-09-10)<br>entire document, in particular description, paragraphs 6-13 | 1-12 |
| A | CN 104045633 A (FUJIAN MINDONG REJUENATION PHARMACEUTICAL CO., LTD.) 17 September 2014 (2014-09-17)<br>entire document | 1-12 |
| A | CN 104059056 A (FUJIAN MINDONG REJUENATION PHARMACEUTICAL CO., LTD.) 24 September 2014 (2014-09-24)<br>entire document | 1-12 |
| A | WO 2013000406 A1 (LIN, Tongjun et al.) 03 January 2013 (2013-01-03)<br>entire document | 1-12 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 August 2023** | **11 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/090820**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:

Claim 13 relates to a method for treating allergic diseases or disorders, i.e., a method for treatment of a human or animal body by therapy, which belongs to subject matter for which a search is not required by the International Searching Authority (PCT Rule 39.1).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/090820**

| Patent document cited in search report | | | Publication date (day/month/year) | Patent family member(s) | | | Publication date (day/month/year) |
|---|---|---|---|---|---|---|---|
| CN | 104031035 | A | 10 September 2014 | CN | 104031035 | B | 25 August 2017 |
| CN | 104045633 | A | 17 September 2014 | CN | 104045633 | B | 21 July 2017 |
| CN | 104059056 | A | 24 September 2014 | CN | 104059056 | B | 25 August 2017 |
| WO | 2013000406 | A1 | 03 January 2013 | US | 2014135361 | A1 | 15 May 2014 |
| | | | | US | 9296731 | B2 | 29 March 2016 |
| | | | | EP | 2727919 | A1 | 07 May 2014 |
| | | | | EP | 2727919 | A4 | 10 December 2014 |
| | | | | EP | 2727919 | B1 | 08 May 2019 |
| | | | | US | 2016166563 | A1 | 16 June 2016 |
| | | | | US | 10022362 | B2 | 17 July 2018 |
| | | | | JP | 2014526438 | A | 06 October 2014 |
| | | | | JP | 5978296 | B2 | 24 August 2016 |
| | | | | CN | 103619839 | A | 05 March 2014 |
| | | | | CN | 103619839 | B | 01 June 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013000406 A
- CN 104045633 B
- CN 104059056 B
- CN 104031035 B